(19) Europäisches Patentamt
European Patent Office
Office européen des brevets

(11) EP 2 104 686 B2

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**27.11.2019 Patentblatt 2019/48**

(45) Hinweis auf die Patenterteilung:
**27.07.2011 Patentblatt 2011/30**

(21) Anmeldenummer: **07848074.6**

(22) Anmeldetag: **12.12.2007**

(51) Int Cl.:
***C08F 2/00*** *(2006.01)* ***C08F 2/10*** *(2006.01)*
***C08F 2/16*** *(2006.01)* ***C08F 2/18*** *(2006.01)*
***A61L 15/60*** *(2006.01)*

(86) Internationale Anmeldenummer:
**PCT/EP2007/063758**

(87) Internationale Veröffentlichungsnummer:
**WO 2008/077780 (03.07.2008 Gazette 2008/27)**

(54) **VERFAHREN ZUR HERSTELLUNG MECHANISCH STABILER WASSERABSORBIERENDER POLYMERPARTIKEL**

METHOD FOR PRODUCING MECHANICALLY STABLE WATER-ABSORBENT POLYMER PARTICLES

PROCÉDÉ DE PRÉPARATION DE PARTICULES POLYMÈRES MÉCANIQUEMENT STABLES QUI ABSORBENT L'EAU

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **22.12.2006 EP 06126998**

(43) Veröffentlichungstag der Anmeldung:
**30.09.2009 Patentblatt 2009/40**

(73) Patentinhaber: **BASF SE**
**67056 Ludwigshafen am Rhein (DE)**

(72) Erfinder:
- **STUEVEN, Uwe**
  **65812 Bad Soden (DE)**
- **FUNK, Rüdiger**
  **65527 Niedernhausen (DE)**
- **WEISMANTEL, Matthias**
  **63637 Jossgrund (DE)**
- **HEIDE, Wilfried**
  **67251 Freinsheim (DE)**
- **KRÜGER, Marco**
  **68161 Mannheim (DE)**

(74) Vertreter: **BASF IP Association**
**BASF SE**
**G-FLP-C006**
**67056 Ludwigshafen (DE)**

(56) Entgegenhaltungen:
**EP-A- 0 703 265** **EP-A- 0 755 964**
**EP-A2- 0 349 241** **EP-A2- 1 029 886**
**WO-A-01/25290** **WO-A-2006/079631**
**WO-A1-2006/077054** **WO-A2-2006/082239**
**WO-A2-2008/040714** **DE-A1- 3 637 057**
**JP-A- H1 171 425** **JP-A- H09 124 879**
**JP-A- H11 147 902** **JP-A- 2006 342 306**
**US-A- 4 742 086** **US-B2- 6 720 073**

Bemerkungen:
Die Akte enthält technische Angaben, die nach dem Eingang der Anmeldung eingereicht wurden und die nicht in dieser Patentschrift enthalten sind.

EP 2 104 686 B2

## Beschreibung

Die vorliegende Erfindung betrifft ein Verfahren zur Herstellung wasserabsorbierender Polymerpartikel durch Polymerisation von Tropfen einer Monomerlösung in einer die Tropfen umgebenden Gasphase und Nachvernetzung der Polymerpartikel, wobei die nachvernetzten Polymerpartikel zumindest teilweise beschichtet werden.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Wasserabsorbierende Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Eigenschaften der wasserabsorbierenden Polymere können über den Vernetzungsgrad eingestellt werden. Mit steigendem Vernetzungsgrad steigt die Gelfestigkeit und sinkt die Absorptionskapazität.

Zur Verbesserung der Anwendungseigenschaften, wie beispielsweise Flüssigkeitsweiterleitung im gequollenen Gelbett (SFC) in der Windel und Absorption unter Druck (AUP), werden wasserabsorbierende Polymerpartikel im allgemeinen nachvernetzt. Dadurch steigt nur der Vernetzungsgrad der Partikeloberfläche, wodurch die Absorption unter Druck (AUP) und die Zentrifugenretentionskapazität (CRC) zumindest teilweise entkoppelt werden können. Diese Nachvernetzung kann in wässriger Gelphase durchgeführt werden. Vorzugsweise werden aber getrocknete, gemahlene und abgesiebte Polymerpartikel (Grundpolymer) an der Oberfläche mit einem Nachvernetzer beschichtet, thermisch nachvernetzt und getrocknet. Dazu geeignete Vernetzer sind Verbindungen, die Gruppen enthalten, die mit den Carboxylatgruppen des hydrophilen Polymeren kovalente Bindungen bilden können.

Durch Sprühpolymerisation konnten die Verfahrensschritte Polymerisation und Trocknung zusammengefasst werden. Zusätzlich konnte die Partikelgröße durch geeignete Verfahrensführung in gewissen Grenzen eingestellt werden.

Die Herstellung wasserabsorbierender Polymerpartikel durch Polymerisation von Tropfen einer Monomerlösung wird beispielsweise in EP 348 180 A1, WO 96/40427 A1, US 5,269,980, DE 103 14 466 A1, DE 103 40 253 A1, DE 10 2004 024 437 A1 und DE 10 2005 002 412 A1 sowie der älteren deutschen Anmeldung mit dem Aktenzeichen 102006001596.7 beschrieben.

DE 10 2004 042 946 A1, DE 10 2004 042 948 A1, DE 10 2004 042 955 A1 und DE 10 2005 019 398 A1 beschreiben die Herstellung von Verdickern durch Sprühpolymerisation.

WO 2006/079631 A1 beschreibt ein Verfahren zur Vertropfungspolymerisation, wobei die Polymerpartikel in einer Wirbelschicht getrocknet und wahlweise nachvernetzt werden.

EP 703 265 A1 beschreibt ein Verfahren zur Verbesserung der Abriebfestigkeit wasserabsorbierender Polymerpartikel durch Beschichtung mit filmbildenden Polymeren.

EP 755 964 A2 offenbart ebenfalls ein Verfahren zur Verbesserung der Abriebfestigkeit wasserabsorbierender Polymerpartikel, wobei die Partikel mit Wachsen beschichtet werden.

Aufgabe der vorliegenden Erfindung war die Bereitstellung eines verbesserten Verfahrens zur Herstellung wasserabsorbierender Polymerpartikel durch Polymerisation von Tropfen einer Monomerlösung in einer die Tropfen umgebenden Gasphase.

Gelöst wurde die Aufgabe durch ein Verfahren zur Herstellung wasserabsorbierender Polymerpartikel durch Polymerisation von Tropfen einer Monomerlösung, enthaltend

1. a) mindestens ein ethylenisch ungesättigtes Monomer,
2. b) wahlweise mindestens einen Vernetzer,
3. c) mindestens einen Initiator,
4. d) Wasser,

in einer die Tropfen umgebenden Gasphase, wobei die erhaltenen Polymerpartikel nachvernetzt werden, dadurch gekennzeichnet, dass die nachvernetzten Polymerpartikel zumindest teilweise beschichtet werden.

Durch Polymerisation von Monomerlösungstropfen in einer die Tropfen umgebenden Gasphase ("Vertropfungspolymerisation") werden runde Polymerpartikel mit hoher mittlerer Sphärizität (mSPHT) erhalten. Die mittlere Sphärizität ist ein Maß für die Rundheit der Polymerpartikel und kann beispielsweise mit dem Bildanalysesystem Camsizer® (Retsch Technolgy GmbH; DE) bestimmt werden.

Der vorliegenden Erfindung liegt die Erkenntnis zugrunde, dass es sich bei den durch Vertropfungspolymerisation erhaltenen Polymerpartikeln um Hohlkugeln handelt und dass die Hohlkugeln durch mechanische Belastung beschädigt werden können.

Durch die erfindungsgemäße Beschichtung kann die mechanische Stabilität der Hohlkugeln deutlich erhöht werden. Geeignete Beschichtungsmittel sind beispielsweise Zusätze, die die Elastizität der Partikeloberfläche erhöhen, beispielsweise durch Absenkung der Glasübergangstemperatur, und/oder die Partikeloberfläche mechanisch verfestigen.

Die Beschichtung kann dadurch hergestellt werden, dass die durch Vertropfungspolymerisation hergestellten wasserabsorbierenden Polymerpartikel in an sich bekannter Weise durch Einmischens des Beschichtungsmittels im gewünschten Gewichtsverhältnis beschichtet werden. Dieses Beschichten geschieht bevorzugt in Mischern mit bewegten Mischwerkzeugen, wie Schneckenmischer, Paddelmischer, Scheibenmischer, Pflugscharmischer und Schaufelmischer. Besonders bevorzugt sind Vertikalmischer, ganz besonders bevorzugt sind Pflugscharmischer und Schaufelmischer. Geeignete Mischer sind beispielsweise Lödige-Mischer, Bepex-Mischer, Nauta-Mischer, Processall-Mischer und Schugi-Mischer. Ganz besonders bevorzugt werden Hochgeschwindigkeitsmischer, beispielsweise vom Typ Schuggi-Flexomix oder Turbolizer eingesetzt. Ganz besonders bevorzugt sind aber auch Wirbelschichtmischer.

Die nachvernetzten wasserabsorbierenden Polymerpartikel werden vorzugsweise mit Wasser, einer wässrigen Lösung, einem Alkanolamin, einem Polymeren und/oder einem Wachs beschichtet.

Die Einsatzmenge an Wasser oder wässriger Lösung bei der erfindungsgemäßen Beschichtung beträgt vorzugsweise 1 bis 30 Gew.-%, besonders bevorzugt 2 bis 20 Gew.-%, ganz besonders bevorzugt 3 bis 15 Gew.-%.

In einer bevorzugten Ausführungsform bei der Beschichtung mit Wasser oder einer wässrigen Lösung ein Tensid als Deagglomerationshilfsmittel, beispielsweise Sorbitanmonoester, wie Sorbitanmonococoat und Sorbitanmonolaurat, oder ethoxylierte Varianten davon zugesetzt. Weitere sehr geeignete Deagglomerationshilfsmittel stellen die ethoxylierten und alkoxylierten Derivate des 2-Propylheptanols dar, die unter den Handelsmarken Lutensol® XL und Lutensol® XP vertrieben werden (BASF Aktiengesellschaft, DE).

Die Einsatzmenge des Deagglomerationshilfsmittels bezogen auf die wasserabsorbierenden Polymerpartikel beträgt beispielsweise von 0,01 bis 1 Gew.-%, vorzugsweise von 0,05 bis 0,5 Gew.-%, besonders bevorzugt von 0,1 bis 0,2 Gew.-%.

Die Einsatzmenge an Alkanolamin bei der erfindungsgemäßen Beschichtung beträgt vorzugsweise 0,5 bis 30 Gew.-%, besonders bevorzugt 1 bis 20 Gew.-%, ganz besonders bevorzugt 2 bis 15 Gew.-%, wobei die Alkanolamine auch als Lösung in einem geeigneten Lösungsmittel, beispielsweise Wasser eingesetzt werden können.

Geeignete Alkanolamine sind Mono-, Di- und Trialkanolamine, wie Ethanolamin, Diethanolamin oder Triethanolamin. Bevorzugte sind tertiäre Alkanolamine, wie Triethanolamin, Methyldiethanolamin, Dimethylaminodiglykol, Dimethylethanolamin und N,N,N',N'-Tetra-(hydroxyethyl)-ethylendiamin. Besonders bevorzugt ist Triethanolamin.

Die Menge an Polymer und/oder Wachs beträgt vorzugsweise 0,005 bis 10 Gew.-%, besonders bevorzugt 0,05 bis 5 Gew.-%, ganz besonders bevorzugt 0,1 bis 2 Gew.-%, jeweils bezogen auf die Polymerpartikel.

Das Polymer kann auch auf die Partikeloberfläche aufgebracht werden, indem die Vorstufen des Polymers auf die Partikeloberfläche aufgebracht werden, die erst auf der Partikeloberfläche zu dem gewünschten Polymer abreagieren, beispielsweise durch Umsetzung von Polyolen mit Polyepoxiden.

Die Polymere, deren Vorstufen oder Wachse, können als wässrige Dispersionen, Emulsionen und/oder Polymersuspensionen auf die Partikeloberfläche aufgebracht werden.

Die Polymere, deren Vorstufen oder Wachse, können aber auch in Form einer Lösung in einem organischen Lösungsmittel oder in einem Gemisch aus Wasser und einem organischen wassermischbaren Lösungsmittel eingesetzt werden. Auch die genannten wässrigen Dispersionen, Emulsionen und Suspensionen können einen Anteil an organischem wahlweise wassermischbaren Lösungsmittel enthalten.

Geeignete organische Lösungsmittel sind beispielsweise aliphatische und aromatische Kohlenwasserstoffe, wie n-Hexan, Cyclohexan, Toluol und Xylol, Alkohole, wie Methanol, Ethanol, Isopropanol, Ethylenglykol, Propylenglykol, Glycerin und Polyethylenglykole mit mittlerem Molekulargewicht von 200 bis 10.000, Ether, wie Diethylether, Ester, wie Ethylacetat und n-Butylacetat, und Ketone, wie Aceton und 2-Butanon.

Geeignete wassermischbare organische Lösungsmittel sind beispielsweise aliphatische $C_1$- bis $C_4$-Alkohole, wie Methanol, Isopropanol, tert.-Butanol, Ethylenglykol, Propylenglykol, Glycerin und Polyethylenglykole mit mittlerem Molekulargewicht von 200 bis 10,000, Ether und Ketone, wie Aceton und 2-Butanon.

Die Polymere und/oder Wachse können aber auch als Schmelze dosiert werden.

Die im erfindungsgemäßen Verfahren einsetzbaren Polymere und/oder Wachse sind vorzugsweise nicht reaktiv, d.h. sie weisen keine reaktiven Gruppen auf, die mit den Gruppen an der Oberfläche der Polymerpartikel reagieren.

Bevorzugte Polymere und/oder Wachse sind außerdem insbesondere solche, die im Temperaturbereich zwischen 0 °C und 80 °C nicht zum Verkleben neigen.

Bevorzugte erfindungsgemäß zur Beschichtung einzusetzende Polymere sind Homo-und Copolymere von Vinylestern, insbesondere Vinylacetat-Homopolymere und Vinylacetat-Copolymere mit Ethylen, Acrylaten, Maleinsäureestern, Vinylamiden und/oder anderen Vinylacylderivaten.

Bevorzugt sind außerdem Homo- und Copolymere von Acryl- und Methacrylsäureestern, wie beispielsweise Copolymere von Methylmethacrylat und Acrylsäure-n-butylester oder Acrylsäure-2-ethylhexylester.

Die genannten Copolymere auf Basis Vinyl-, Acrylsäure- und Methacrylsäureestern können als weitere Comonomere beispielsweise Styrol, Butadien, Vinylamide, olefinisch ungesättigte Carbonsäuren und deren Derivate, olefinisch ungesättigte Sulfonsäuren und deren Derivate, Vinylphosphonsäure und deren Derivate oder Polyglykolester ungesättigter Säuren enthalten.

Beispiele für Vinylamide sind insbesondere N-Vinylformamid, N-Vinyl-N-methylacetamid und N-Vinylpyrrolidon.

Beispiele für olefinisch ungesättigte Carbonsäuren sind insbesondere Acrylsäure, Methacrylsäure, Itaconsäure und Maleinsäure sowie deren Alkali-, Ammonium- und Aminsalze. Beispiele für Derivate dieser olefinisch ungesättigten Carbonsäuren sind insbesondere Amide, wie (Meth)acrylamid, N-tert.-Butyl(meth)acrylamid und N-Isopropyl(meth)acrylamid, aber auch N-Methylolamide oder Ether der N-Methylolamide, Halbamide und Imide aliphatischer Amine, sowie Acrylnitril.

Beispiele für olefinisch ungesättigte Sulfonsäuren sind die Salze der Vinylsulfonsäure, 2-Acrylamido-2-methylpropansulfonsäure, Styrolsulfonsäure, Allyl- und Methallylsulfonsäure, insbesondere deren Alkali-, Ammonium- und Aminsalze.

Beispiele für Derivate der Vinylphosphonsäure sind insbesondere die Mono- und Diester von $C_1$- bis $C_{18}$-Alkoholen, wie beispielsweise die Methyl-, Propyl- oder Stearylester. Die Vinylphosphonsäure selbst liegt insbesondere als Mono- oder Disalz vor, wobei die Alkali-, Ammonium- und Aminsalze bevorzugt sind.

Polyglykolester ungesättigter Säuren sind insbesondere Hydroxyethyl(meth)acrylat oder Ester der Acryl- und Methacrylsäure mit Polyalkylenoxidverbindungen der allgemeinen Formel

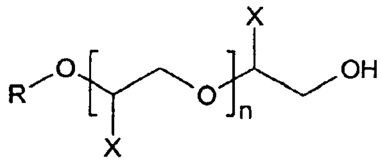

wobei

X Wasserstoff oder Methyl,

n 0 bis 50 und

R einen aliphatischen, araliphatischen oder cycloaliphatischen $C_1$- bis $C_{24}$-Rest, beispielsweise Nonylphenyl, bedeuten.

In einer bevorzugten Ausführungsform der vorliegenden Erfindung werden filmbildende Polymere zur Beschichtung verwendet. Dies sind insbesondere Polymere, die Polymerfilme mit einer Reißfestigkeit von 0,5 bis 25 N/mm, vorzugsweise von 1 bis 20 N/mm, besonders bevorzugt von 2 bis 15, ganz besonders bevorzugt von 5 bis 10 N/mm, und eine Reißdehnung von 10 bis 10.000 %, vorzugsweise von 20 bis 5.000 %, besonders bevorzugt von 50 bis 2.000 %, ganz besonders bevorzugt von 500 bis 1.000 %, aufweisen. Die Reißfestigkeit und die Reißdehnung werden gemäß DIN EN ISO 527 bestimmt.

Bevorzugte erfindungsgemäß zur Beschichtung einzusetzende Polymere sind weiterhin filmbildende Polymere auf Basis von

- Polyacetalen, d.h. Umsetzungsprodukten von Polyvinylalkoholen mit Aldehyden, wie Butyraldehyd,
- Polyurethanen, d.h. aus durch Polyaddition aus zwei- und höherwertigen Alkoholen und Isocyanaten zugänglichen Polymeren, beispielsweise hergestellt aus Polyester- und/oder Polyetherdiolen und zum Beispiel 2,4- bzw. 2,6-Toluoldiisocyanat, 4,4-Methylendi(phenylisocyanat) oder Hexamethylendiisocyanat (siehe Houben-Weyl, Methoden der Organischen Chemie, 4. Auflage, Band E20/2, Seiten 1561 bis 1721),
- Polyharnstoffen, d.h. Polymeren, die zugänglich sind durch Polyaddition von Diaminen und Diisocyanaten oder durch Polykondensation von Diaminen mit Kohlenstoffdioxid, Phosgen, Carbonsäureestern (z.B. aktivierte Diphenylcarbonate) oder Harnstoff bzw. durch Umsetzung von Diisocyanaten mit Wasser (siehe Houben-Weyl, Methoden der Organischen Chemie, 4. Auflage, Band E20/2, Seiten 1721 bis 1752),
- Polysiloxanen, wobei als Basispolymer insbesondere lineares Dimethylpolysiloxan verwendet wird, dessen Endgruppen unterschiedlich modifiziert sein können (siehe "Chemie und Technologie des kalthärtenden Siliconkautschuks", Seiten 49 bis 64 in SILICONE - Chemie und Technologie, [Symposium am 28.04.1989] VULKAN-VERLAG, Essen),
- Polyamiden, wobei Copolyamide (siehe Plaste Kautsch. Jahrgang 25, Seiten 440 bis 444 (1978)), wie sie beispielsweise zur Herstellung von Lacken Verwendung finden, bevorzugt sind.
- Polyestern, d.h. Polymeren, die durch Ringöffnungspolymerisation von Lactonen oder durch Polykondensation von Hydroxycarbonsäuren bzw. von Diolen und Dicarbonsäurederivaten hergestellt werden (siehe Houben-Weyl, Methoden der Organischen Chemie, 4. Auflage, Band E20/2, Seiten 1404 bis 1429),

- Epoxidharzen, die aus Polyepoxiden durch Polyadditionsreaktionen mit geeigneten Härtern oder durch Polymerisation über Epoxidgruppen hergestellt werden können (siehe Houben-Weyl, Methoden der Organischen Chemie, 4. Auflage, Band 14/2, Seiten 462 bis 552 und Band E20/2, Seiten 1891 bis 1994 (z.B. Umsetzungsprodukte aus Bisphenol A mit Epichlorhydrin) oder auf Basis von
- Polycarbonaten, wie sie leicht durch Umsetzung von Diglykolen oder Bisphenolen mit Phosgen bzw. Kohlensäurediestern in Polykondensations- bzw. Umesterungsreaktionen herzustellen sind (siehe Houben-Weyl, Methoden der Organischen Chemie, 4. Auflage, Band E 20/2, Seiten 1443 bis 1457).

Besonders bevorzugte erfindungsgemäß zur Beschichtung einzusetzende Polymere sind Homo- und Copolymere von Acryl- und Methacrylsäureestern sowie Polymere auf Basis von Polyacetalen.

Es können auch Mischungen zweier oder mehrerer der oben genannten Polymere eingesetzt werden. Die Mischungsverhältnisse sind dabei vollkommen unkritisch und den jeweiligen Gegebenheiten anzupassen.

Bevorzugte im erfindungsgemäßen Verfahren einzusetzende Polymere sind weiterhin elastische Polymere. Dies sind Polymere mit gummielastischem Verhalten, die bei 23 °C wiederholt mindestens auf das Zweifache ihrer Länge gedehnt werden können und nach Aufhebung des für die Dehnung erforderlichen Zwanges sofort wieder annähernd ihre Ausgangslänge annehmen.

Repräsentative Beispiele für geeignete elastische Polymere sind natürliche und synthetische Latices, die üblicherweise als Bindemittel und elastomere Kleber bei der Herstellung von lufttrocknenden absorbierenden Produkten verwendet werden. Zusätzlich zu den in den Beispielen beschriebenen Latices wird in Betracht gezogen, dass jedes zur Bildung einer Latexdispersion geeignete natürliche oder synthetische Elastomer zur Verwendung in der vorliegenden Erfindung geeignet ist. Daher können natürlicher Gummi, Polybutadien-Gummi, Styrol-Butadien-Gummi, Arylnitril-Butadien-Gummi, Poly-2-chlorbutadien-Gummi, Polyisopren-Gummi, Isopren-Isobutylen-Copolymere, Ethylen-Propylen-Gummi, Ethylen-Vinylacetat-Copolymere, chloriertes Polyethylen, chlorsulfoniertes Polyethylen, Acryl-Gummi, Ethylen-Acrylat-Copolymere, Epichlorhydrin-Gummi, Polypropylenoxid-Gummi und Polyurethane verwendet werden.

In einer weiteren bevorzugten Ausführungsform der vorliegenden Erfindung werden Wachse auf die Oberfläche der nachvernetzten wasserabsorbierenden Polymerpartikel aufgebracht.

Unter einem Wachs ist dabei insbesondere, entsprechend der Formulierung der Deutschen Gesellschaft für Fettwissenschaft (DGF) von 1974 (siehe DGF-Einheitsmethoden: Untersuchung von Fetten, Fettprodukten und verwandten Stoffen, Abteilung M: Wachse und Wachsprodukte; Wissenschaftliche Verlagsgesellschaft, Stuttgart, 1975), eine Substanz zu verstehen, die unabhängig von ihrer chemischen Zusammensetzung und ihrer natürlichen oder künstlichen Herkunft in der Regel durch folgende mechanisch-physikalische Eigenschaften gekennzeichnet ist:

- bei 20°C knetbar, fest bis brüchig hart;
- grob- bis feinkristallin, durchscheinend bis opak, jedoch nicht glasartig;
- über 40°C ohne Zersetzung schmelzend;
- schon wenig oberhalb des Schmelzpunktes verhältnismäßig niedrigviskos und nicht fadenziehend;
- stark temperaturabhängig in Konsistenz und Löslichkeit;
- unter leichtem Druck polierbar.

Bevorzugte Wachse sind insbesondere solche, deren Schmelz- bzw. Tropfpunkte im Temperaturbereich zwischen 30 und 180 °C, besonders bevorzugt zwischen 40 und 180 °C, ganz besonders bevorzugt zwischen 40 und 170 °C, liegen. Die Bestimmung des Tropfpunktes erfolgt dabei nach der DGF-Einheitsmethode DGF-M-III 3 (75) (Wissenschaftliche Verlagsgesellschaft, Stuttgart).

Erfindungsgemäß einzusetzende Wachse sind beispielsweise Naturwachse, modifizierte Naturwachse, teilsynthetische Wachse und vollsynthetische Wachse. Beispiele für Naturwachse sind rezente Wachse, wie Pflanzenwachse oder Tierwachse. Beispiele für Pflanzenwachse sind Carnaubawachs, Candelillawachs, Ouricuriwachs, Zuckerrohrwachs und Retamowachs. Beispiele für Tierwachse sind Insektenwachse, wie Bienenwachs, Gheddawachs und Schellackwachs, sowie Wollwachs. Weitere Beispiele für Naturwachse sind fossile Wachse, wie Erdölwachse oder Braunkohlen- und Torfwachse. Beispiele für Erdölwachse sind Ozokerit und Tankbodenwachs, ein Beispiel für ein Braunkohlen- und Torf- wachs ist Rohmontanwachs. Beispiele für modifizierte Naturwachse sind die durch Raffination erhaltenen Wachse, wie die aus Erdöldestillaten bzw. Destillatrückständen gewonnenen makro- und mikrokristallinen Paraffinwachse oder chemisch veränderte Wachse, wie doppelt gebleichtes Rohmontanwachs. Beispiele für teilsynthetische Wachse sind die aus Montanwachs herstellbaren Säurewachse und Esterwachse, die durch Paraffin-Oxidation herstellbaren Wachssäuren sowie Alkoholwachse und Amidwachse. Beispiele für vollsynthetische Wachse sind Kohlenwasserstoff-Wachse, wie Polyolefinwachse und Fischer-Tropsch-Wachse, sowie Synthesewachse mit sauerstofffunktionellen Gruppen. Beispiele für Synthesewachse mit sauerstofffunktionellen Gruppen sind Säurewachse, die durch Oxidation von synthetisch hergestellten Kohlenwasserstoff-Wachsen oder durch Copolymerisation bzw. Telomerisation von Olefinen mit ungesättigten Carbonsäuren entstehen, Esterwachse, die durch Veresterung synthetischer Wachssäuren mit synthetischen Alkoholen und durch Copolymerisation von Olefinen mit ungesättigten Estern, wie Vinylacetat, erhalten werden, Alkoholwachse, die durch Oxosynthese mit anschließender Hydrierung sowie durch Hydrierung synthetischer Fettsäuren hergestellt werden, sowie Amidwachse, die durch Umsetzung synthetischer Säuren mit Aminen erhalten werden. Beispiele für Wachse, die durch Oxidation von synthetisch hergestellten Kohlenwasserstoff-Wachsen erhalten werden, sind Oxidate von Polyethylenwachsen.

Bevorzugte erfindungsgemäß einzusetzende Wachse sind veredelte (d.h. entharzte und gebleichte) Montanwachse sowie Polyolefinwachse.

Besonders bevorzugte erfindungsgemäß einzusetzende Wachse sind Polyolefinwachse, wie Polyethylenwachse (Hochdruck-Polyethylenwachse, Niederdruck-Polyethylenwachse, Abbau-Polyethylenwachse), Oxidate dieser Polyethylenwachse, Wachse basierend auf Ethen-α-Olefin-Copolymeren, Wachse basierend auf Ethylen-Vinylacetat-Copolymeren, Wachse, basierend auf Ethylen-Styrol-Copolymeren, Wachse, basierend auf Ethylen-Acrylsäure-Copolymeren, sowie Wachse, basierend auf Wachsmischungen von Polyethylenwachsen mit Poly(tetrafluorethylen)wachsen.

Es können auch Mischungen zweier oder mehrerer der oben genannten Wachse eingesetzt werden. Die Mischungsverhältnisse sind dabei vollkommen unkritisch und den jeweiligen Gegebenheiten anzupassen.

Die im erfindungsgemäßen Verfahren einzusetzenden Monomerlösungen zur Herstellung der wasserabsorbierenden Polymerpartikel enthalten mindestens ein ethylenisch ungesättigtes Monomer a), wahlweise mindestens einen Vernetzer b), mindestens einen Initiator c) und Wasser d).

Die Monomeren a) sind vorzugsweise wasserlöslich, d.h. die Löslichkeit in Wasser bei 23 °C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 50 g/100 g Wasser, und haben vorzugsweise mindestens je eine Säuregruppe.

Geeignete Monomere a) sind beispielsweise ethylenisch ungesättigte Carbonsäuren, wie Acrylsäure, Methacrylsäure, Maleinsäure, Fumarsäure und Itaconsäure. Besonders bevorzugte Monomere sind Acrylsäure und Methacrylsäure. Ganz besonders bevorzugt ist Acrylsäure.

Die bevorzugten Monomere a) haben mindestens eine Säuregruppe, wobei die Säuregruppen vorzugsweise zumindest teilweise neutralisiert sind.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Säuregruppen der Monomere a) sind üblicherweise teilweise neutralisiert, vorzugsweise zu 25 bis 85 mol-%, bevorzugt zu 50 bis 80 mol-%, besonders bevorzugt 60 bis 75 mol-%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen. Üblicherweise wird die Neutralisation durch Einmischung des Neutralisationsmittels als wässrige Lösung, als Schmelze, oder bevorzugt auch als Feststoff erreicht. Beispielsweise kann Natriumhydroxid mit einem Wasseranteil deutlich unter 50 Gew.-% als wachsartige Masse mit einem Schmelzpunkt oberhalb 23 °C vorliegen. In diesem Fall ist eine Dosierung als Stückgut oder Schmelze bei erhöhter Temperatur möglich.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden

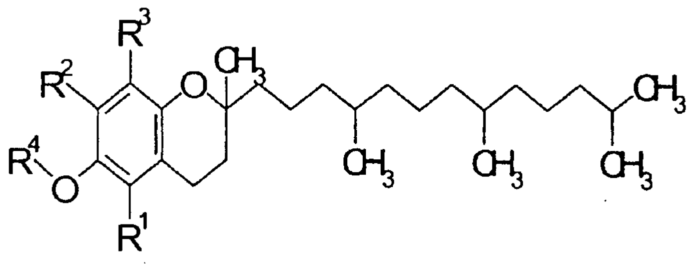

wobei $R^1$ Wasserstoff oder Methyl, $R^2$ Wasserstoff oder Methyl, $R^3$ Wasserstoff oder Methyl und $R^4$ Wasserstoff oder ein Säurerest mit 1 bis 20 Kohlenstoffatomen bedeutet.

Bevorzugte Reste für $R^4$ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit $R^1 = R^2 = R^3 =$ Methyl, insbesondere racemisches alpha-Tocopherol. $R^1$ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindesten 10 Gew.-ppm, besonders bevorzugt mindesten 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die Polymerisationsinhibitoren können auch durch Absorption, beispielsweise an Aktivkohle, aus der Monomerlösung entfernt werden.

Vernetzer b) sind Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP 530 438 A1 beschrieben, Di- und Triacrylate, wie in EP 547 847 A1, EP 559 476 A1, EP 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/32962 A2 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP 343 427 A2 beschrieben sind. Weiterhin geeignete Vernetzer b) sind Pentaerythritoldi-, Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 100 und 1000 aufweist.

Besonders vorteilhafte Vernetzer b) sind jedoch Di- und Triacrylate des 3- bis 20-fach ethoxylierten Glyzerins, des 3- bis 20-fach ethoxylierten Trimethylolpropans, des 3- bis 20-fach ethoxylierten Trimethylolethans, insbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des mindestens 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3-bis 5-fach ethoxylierten und/oder propoxylierten Glyzerins.

Die Monomerlösung enthält vorzugsweise mindestens 0,1 Gew.-%, bevorzugt mindestens 0,2 Gew.-%, besonders bevorzugt mindestens 0,3 Gew.-%, ganz besonders bevorzugt mindestens 0,4 Gew.-%, und vorzugsweise bis zu 2,5 Gew.-%, bevorzugt bis zu 2 Gew.-%, besonders bevorzugt bis zu 1,5 Gew.-%, ganz besonders bevorzugt bis zu 1 Gew.-%, Vernetzer b), jeweils bezogen auf Monomer a).

Als Initiatoren c) können sämtliche unter den Polymerisationsbedingungen in Radikale zerfallende Verbindungen eingesetzt werden, beispielsweise Peroxide, Hydroperoxide, Wasserstoffperoxid, Persulfate, Azoverbindungen und die sogenannten Redoxinitiatoren. Bevorzugt ist der Einsatz von wasserlöslichen Initiatoren. In manchen Fällen ist es vorteilhaft, Mischungen verschiedener Initiatoren zu verwenden, beispielsweise Mischungen aus Wasserstoffperoxid und Natrium- oder Kaliumperoxodisulfat. Mischungen aus Wasserstoffperoxid und Natriumperoxodisulfat können in jedem beliebigen Verhältnis verwendet werden.

Besonders bevorzugte Initiatoren c) sind Azoinitiatoren, wie 2,2'-Azobis[2-(2-imidazolin-2-yl)propan]dihydrochlorid und 2,2'-Azobis[2-(5-methyl-2-imidazolin-2-yl)propan]dihydrochlorid, und Photoinitiatoren, wie 2-Hydroxy-2-methylpropiophenon und 1-[4-(2-Hydroxyethoxy)-phenyl]-2-hydroxy-2-methyl-1-propan-1-on, Redoxinitiatoren, wie Natriumpersulfat/ Hydroxymethylsulfinsäure, Ammoniumperoxodisulfat/Hydroxymethylsulfinsäure, Wasserstoffperoxid/Hydroxymethylsulfinsäure, Natriumpersulfat/Ascorbinsäure, Ammoniumperoxodisulfat/Ascorbinsäure und Wasserstoffperoxid/Ascorbinsäure, Photoinitiatoren, wie 1-[4-(2-Hydroxyethoxy)-phenyl]-2-hydroxy-2-methyl-1-propan-1-on, sowie deren Mischungen.

Die Initiatoren werden in üblichen Mengen eingesetzt, beispielsweise in Mengen von 0,001 bis 5 Gew.-%, vorzugsweise 0,01 bis 1 Gew.-%, bezogen auf die Monomeren a).

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt.

Die Monomerlösung wird zur Polymerisation in der Gasphase vertropft.

Der Feststoffgehalt der Monomerlösung beträgt vorzugsweise mindestens 35 Gew.-%, bevorzugt mindestens 38 Gew.-%, besonders bevorzugt mindestens 40 Gew.-%, ganz besonders bevorzugt mindestens 42 Gew.-%. Dabei ist der Feststoffgehalt die Summe aller nach der Polymerisation nichtflüchtigen Bestandteile. Dies sind Monomer a), Vernetzer b) und Initiator c).

Der Sauerstoffgehalt der Gasphase beträgt vorzugsweise 0,001 bis 0,15 Vol.-%, besonders bevorzugt 0,002 bis 0,1 Vol.-%, ganz besonders bevorzugt 0,005 bis 0,05 Vol.-%.

Die Gasphase enthält neben Sauerstoff vorzugsweise nur inerte Gase, d.h. Gase, die unter Reaktionsbedingungen nicht in die Polymerisation eingreifen, beispielsweise Stickstoff und/oder Wasserdampf.

Bei der Vertropfung wird eine Monomerlösung unter Ausbildung von Tropfen in die Gasphase dosiert. Die Vertropfung der Monomerlösung kann beispielsweise mittels einer Vertropferplatte durchgeführt werden.

Eine Vertropferplatte ist eine Platte mit mindestens einer Bohrung, wobei die Flüssigkeit von oben durch die Bohrung tritt. Die Vertropferplatte bzw. die Flüssigkeit kann in Schwingungen versetzt werden, wodurch an der Unterseite der Vertropferplatte je Bohrung eine idealerweise monodisperse Tropfenkette erzeugt wird.

Die Anzahl und die Größe der Bohrungen werden gemäß der gewünschten Kapazität und Tropfengröße ausgewählt. Der Tropfendurchmesser beträgt dabei üblicherweise das 1,9fache des Durchmessers der Bohrung. Wichtig ist hierbei, dass die zu vertropfende Flüssigkeit nicht zu schnell durch die Bohrung tritt bzw. der Druckverlust über die Bohrung nicht zu groß ist. Ansonsten wird die Flüssigkeit nicht vertropft, sondern der Flüssigkeitsstrahl wird infolge der hohen kinetischen Energie zerrissen (versprüht). Der Vertropfer wird im Strömungsbereich des laminaren Strahlzerfalls betrieben, d.h. die Reynoldszahl bezogen auf den Durchsatz pro Bohrung und den Bohrungsdurchmesser ist vorzugsweise kleiner als 2.000, bevorzugt kleiner 1.000, besonders bevorzugt kleiner 500, ganz besonders bevorzugt kleiner 100. Der Druckverlust über die Bohrung beträgt vorzugsweise weniger als 2,5 bar, besonders bevorzugt weniger als 1,5 bar, ganz besonders bevorzugt weniger als 1 bar.

Die Vertropferplatte weist üblicherweise mindestens eine, vorzugsweise mindestens 10, besonders bevorzugt mindestens 50, und üblicherweise bis zu 10.000, vorzugsweise bis zu 5.000, besonders bevorzugt bis zu 1.000, Bohrungen auf, wobei die Bohrungen üblicherweise gleichmäßig über die Vertropferplatte verteilt sind, vorzugsweise in der sogenannten Dreiecksteilung, d.h. jeweils drei Bohrungen bilden die Ecken eines gleichseitigen Dreiecks.

Der Durchmesser der Bohrungen wird an die gewünschte Tropfengröße angepasst. Die erzeugten Tropfen weisen eine mittlere Tropfengröße von vorzugsweise mindestens 100 μm, besonders bevorzugt von mindestens 150 μm, ganz besonders bevorzugt von mindestens 200 μm, auf, wobei der Tropfendurchmesser durch Lichtstreuung bestimmt werden kann.

Es kann vorteilhaft sein die Vertropferplatte auf eine Trägerplatte aufzulegen, wobei die Trägerplatte ebenfalls Bohrungen aufweist. Dabei weisen die Bohrungen der Trägerplatte einen größeren Durchmesser auf als die Bohrungen der Vertropferplatte auf und sind so angeordnet, dass sich unter jeder Bohrung der Vertropferplatte eine mit ihr konzentrische Bohrung der Trägerplatte befindet. Diese Anordnung ermöglicht einen schnellen Wechsel der Vertropferplatte, beispielsweise um Tropfen einer anderen Größe zu erzeugen.

Die Vertropfung kann aber auch mittels pneumatischer Ziehdüsen, Rotation, Zerschneiden eines Strahls oder schnell ansteuerbarer Mikroventildüsen durchgeführt werden.

In einer pneumatischen Ziehdüse wird ein Flüssigkeitsstrahl zusammen mit einem Gasstrom durch eine Blende beschleunigt. Über die Gasmenge kann der Durchmesser des Flüssigkeitsstrahls und damit der Tropfendurchmesser beeinflusst werden.

Bei der Vertropfung durch Rotation tritt die Flüssigkeit durch die Öffnungen einer rotierenden Scheibe. Durch die auf die Flüssigkeit wirkende Fliehkraft werden Tropfen definierter Größe abgerissen. Bevorzugte Vorrichtungen zur Rotationsvertropfung werden beispielsweise in DE 43 08 842 A1 beschrieben.

Der austretende Flüssigkeitsstrahl kann aber auch mittels eines rotierenden Messers in definierte Segmente zerschnitten werden. Jedes Segment bildet anschließend einen Tropfen.

Bei Verwendung von Mikroventildüsen werden direkt Tropfen mit definiertem Flüssigkeitsvolumen erzeugt.

Bevorzugt strömt die Gasphase als Trägergas durch den Reaktionsraum. Dabei kann das Trägergas im Gleichstrom oder im Gegenstrom zu den frei fallenden Tropfen der Monomerlösung durch den Reaktionsraum geführt werden, bevorzugt im Gleichstrom. Vorzugsweise wird das Trägergas nach einem Durchgang zumindest teilweise, bevorzugt zu mindestens 50 %, besonders bevorzugt zu mindestens 75 %, als Kreisgas in den Reaktionsraum zurückgeführt. Üblicherweise wird eine Teilmenge des Trägergases nach jedem Durchgang ausgeschleust, vorzugsweise bis zu 10 %, besonders bevorzugt bis zu 3 %, ganz besonders bevorzugt bis zu 1 %.

Die Gasgeschwindigkeit wird vorzugsweise so eingestellt, dass die Strömung im Reaktor gerichtet ist, beispielsweise liegen keine der allgemeinen Strömungsrichtung entgegengesetzte Konvektionswirbel vor, und beträgt beispielsweise 0,01 bis 5 m/s, vorzugsweise 0,02 bis 4 m/s, besonders bevorzugt 0,05 bis 3 m/s, ganz besonders bevorzugt 0,1 bis 2 m/s.

Das Trägergas wird zweckmäßigerweise vor dem Reaktor auf die Reaktionstemperatur vorgewärmt.

Die Reaktionstemperatur beträgt bei der thermisch induzierten Polymerisation vorzugsweise 70 bis 250 °C, besonders bevorzugt 100 bis 220 °C, ganz besonders bevorzugt 120 bis 200 °C.

Die Reaktion kann im Überdruck oder im Unterdruck durchgeführt werden, ein Unterdruck von bis zu 100 mbar gegenüber dem Umgebungsdruck ist bevorzugt.

Das Reaktionsabgas, d. h. das der Reaktionsraum verlassende Trägergas, kann beispielsweise in einem Wärmeaustauscher abgekühlt werden. Dabei kondensieren Wasser und nicht umgesetztes Monomer a). Danach kann das Reaktionsabgas zumindest teilweise wieder aufgewärmt und als Kreisgas in den Reaktor zurückgeführt werden. Ein Teil des Reaktionsabgases kann ausgeschleust und durch frisches Trägergas ersetzt werden, wobei im Reaktionsabgas enthaltenes Wasser und nicht umgesetzte Monomere a) abgetrennt und rückgeführt werden können.

Besonders bevorzugt ist ein Wärmeverbund, dass heißt, ein Teil der Abwärme beim Abkühlen des Abgases wird zum Aufwärmen des Kreisgases verwendet.

Die Reaktoren können begleitbeheizt werden. Die Begleitheizung wird dabei so eingestellt, dass die Wandtemperatur mindestens 5 °C oberhalb der Reaktorinnentemperatur liegt und die Kondensation an den Reaktorwänden zuverlässig vermieden wird.

Das Reaktionsprodukt kann dem Reaktor in üblicher Weise entnommen werden, vorzugsweise am Boden über eine Förderschnecke, und gegebenenfalls bis zur gewünschten Restfeuchte und zum gewünschten Restmonomerengehalt getrocknet werden.

Die Polymerpartikel werden zur weiteren Verbesserung der Eigenschaften nachvernetzt. Geeignete Nachvernetzer sind Verbindungen, die mindestens zwei Gruppen enthalten, die mit den Carboxylatgruppen des Hydrogels kovalente Bindungen bilden können. Geeignete Verbindungen sind beispielsweise Alkoxysiliylverbindungen, Polyaziridine, Polyamine, Polyamidoamine, Di- oder Polyepoxide, wie in EP 83 022 A2, EP 543 303 A1 und EP 937 736 A2 beschrieben, di- oder polyfunktionelle Alkohole, wie in DE 33 14 019 A1, DE 35 23 617 A1 und EP 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/31482 A1 Morpholin-2,3-dion und dessen Derivate als geeignete Nachvernetzer beschrieben.

Weiterhin können auch Nachvernetzer eingesetzt werden, die zusätzliche polymerisierbare ethylenisch ungesättigte Gruppen enthalten, wie in DE 37 13 601 A1 beschrieben

Die Menge an Nachvernetzer beträgt vorzugsweise 0,01 bis 1 Gew.-%, besonders bevorzugt 0,05 bis 0,5 Gew.-%, ganz besonders bevorzugt 0,1 bis 0,2 Gew.-%, jeweils bezogen auf das Polymer.

In einer bevorzugten Ausführungsform der vorliegende Erfindung werden zusätzlich zu den Nachvernetzern polyvalente Kationen auf die Partikeloberfläche aufgebracht. Die im erfindungsgemäßen Verfahren einsetzbaren polyvalenten Kationen sind beispielsweise zweiwertige Kationen, wie die Kationen von Zink, Magnesium, Kalzium und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat ist bevorzugt.

Die Einsatzmenge bezogen auf die Polymerpartikel beträgt beispielsweise 0,001 bis 0,5 Gew.-%, vorzugsweise 0,005 bis 0,2 Gew.-%, besonders bevorzugt 0,02 bis 0,1 Gew.-%.

Die Nachvernetzung wird üblicherweise so durchgeführt, dass eine Lösung des Nachvernetzers auf das Hydrogel oder die trockenen Polymerpartikel aufgesprüht wird. Im Anschluss an das Aufsprühen wird thermisch getrocknet, wobei die Nachvernetzungsreaktion sowohl vor als auch während der Trocknung stattfinden kann.

Das Aufsprühen einer Lösung des Vernetzers wird vorzugsweise in Mischern mit bewegten Mischwerkzeugen, wie Schneckenmischer, Paddelmischer, Scheibenmischer, Pflugscharmischer und Schaufelmischer, durchgeführt werden. Besonders bevorzugt sind Vertikalmischer, ganz besonders bevorzugt sind Pflugscharmischer und Schaufelmischer. Geeignete Mischer sind beispielsweise Lödige-Mischer, Bepex-Mischer, Nauta-Mischer, Processall-Mischer und Schugi-Mischer.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex-Trockner und Nara-Trockner. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250 °C, bevorzugt 120 bis 220 °C, und besonders bevorzugt 140 bis 200 °C. Die bevorzugte Verweilzeit bei dieser Temperatur im Reaktionsmischer oder Trockner

beträgt vorzugsweise mindestens 10 Minuten, besonders bevorzugt mindestens 20 Minuten, ganz besonders bevorzugt mindestens 30 Minuten.

Das erfindungsgemäße Verfahren ermöglicht die Herstellung wasserabsorbierender Polymerpartikel mit einer hohen Zentrifugenretentionskapazität (CRC), einer hohen Absorption unter einem Druck von 4,83 kPa (AUP0.7psi) und hoher mechanischer Stabilität.

Die gemäß dem erfindungsgemäßen Verfahren erhältlichen wasserabsorbierenden Polymerpartikel haben üblicherweise die Form von Hohlkugeln. Ein weiterer Gegenstand der vorliegenden Erfindung sind daher wasserabsorbierende Polymerpartikel, enthaltend mindestens einen Hohlraum im Partikelinneren und mindestens 1 Gew.-% Wasser und/oder mindestens ein Alkanolamin, wobei die Vernetzungsdichte an der Partikeloberfläche erhöht wurde.

Der Wassergehalt der erfindungsgemäßen Polymerpartikel beträgt vorzugsweise 1 bis 30 Gew.-%, besonders bevorzugt 2 bis 20 Gew.-%, ganz besonders bevorzugt 3 bis 15 Gew.-%.

Die erfindungsgemäßen Polymerpartikel enthalten vorzugsweise 0,5 bis 30 Gew.-%, besonders bevorzugt 1 bis 20 Gew.-%, ganz besonders bevorzugt 2 bis 15 Gew.-%, mindestens eines Alkanolamins.

Die erfindungsgemäßen wasserabsorbierenden Polymerpartikel sind annähernd rund, d.h. die Polymerpartikel weisen eine mittlere Sphärizität (mSPHT) von typischerweise mindestens 0,84, vorzugsweise mindestens 0,86, besonders bevorzugt mindestens 0,88, ganz besonders bevorzugt mindestens 0,9, auf. Die Sphärizität (SPHT) ist definiert als

$$SPHT = \frac{4\pi A}{U^2},$$

wobei A die Querschnittsfläche und U der Querschnittsumfang der Polymerpartikel ist. Die mittlere Sphärizität (mSPHT) ist die volumengemittelte Sphärizität.

Die mittlere Sphärizität (mSPHT) kann beispielsweise mit dem Bildanalysesystem Camsizer® (Retsch Technolgy GmbH; DE) bestimmt werden.

Polymerpartikel mit relativ niedriger mittlerer Sphärizität (mSPHT) werden durch umgekehrte Suspensionspolymerisation erhalten, wenn die Polymerpartikel während oder nach der Polymerisation agglomeriert werden.

Die durch übliche Lösungspolymerisation (Gelpolymerisation) hergestellten wasserabsorbierende Polymerpartikel werden nach der Trocknung gemahlen und klassiert wobei unregelmäßige Polymerpartikel erhalten werden. Die mittlere Sphärizität (mSPHT) dieser Polymerpartikel beträgt zwischen ca. 0,72 und ca. 0,78.

Ein weiterer Gegenstand der vorliegenden Erfindung sind wasserabsorbierende Polymerpartikel, enthaltend mindestens einen Hohlraum im Partikelinneren, wobei die Vernetzungsdichte an der Partikeloberfläche erhöht wurde, die Partikel eine mittlere Sphärizität (mSPHT) von mindestens 0,84, vorzugsweise von mindestens 0,86, besonders bevorzugt von mindestens 0,88, ganz besonders bevorzugt von mindestens 0,9, und die Partikel einen Absorptions-Index (AUP0.7psi-Index) von mindestens 60 %, vorzugsweise von mindestens 70 %, besonders bevorzugt von mindestens 80 %, ganz besonders bevorzugt von mindestens 85 %, aufweisen.

Ein weiterer Gegenstand der vorliegenden Erfindung sind wasserabsorbierende Polymerpartikel, enthaltend mindestens einen Hohlraum im Partikelinneren, wobei die Vernetzungsdichte an der Partikeloberfläche erhöht wurde, die Polymerpartikel einen mittleren Durchmesser von mindestens 200 $\mu$m und 90% der Polymerpartikel einen Durchmesser von 100 bis 800 $\mu$m aufweisen, eine mittlere Sphärizität (mSPHT) von mindestens 0,84, vorzugsweise von mindestens 0,86, besonders bevorzugt von mindestens 0,88, ganz besonders bevorzugt von mindestens 0,9, und einen Stabilitäts-Index von weniger als 0,15, vorzugsweise von weniger als 0,10, besonders bevorzugt von weniger als 0,075, ganz besonders bevorzugt von weniger als 0,05, aufweisen.

Der Wassergehalt der wasserabsorbierenden Polymerpartikel beträgt typischerweise weniger als 10 Gew.-%, vorzugsweise von weniger als 8 Gew.-%, besonders bevorzugt von weniger als 6 Gew.-%, ganz besonders bevorzugt von weniger als 4 Gew.-%, wobei der Wassergehalt gemäß der von der EDANA (European Disposables and Nonwovens Association) empfohlenen Testmethode Nr. WSP 230.2-05 "Moisture content" bestimmt wird.

Das Verhältnis von maximalem Durchmesser des Hohlraumes zu maximalem Durchmesser des Polymerpartikels beträgt vorzugsweise mindestens 0,1, besonders bevorzugt mindesten 0,3, ganz besonders bevorzugt mindestens 0,4.

Die Polymerpartikel enthalten vorzugsweise mindestens 50 mol-%, besonders bevorzugt mindestens 90 mol-%, ganz besonders bevorzugt mindestens 95 mol-%, polymerisierte Acrylsäure. Die polymerisierte Acrylsäure ist vorzugsweise zu 25 bis 85 mol-%, besonders bevorzugt zu 50 bis 80 mol-%, ganz besonders bevorzugt zu 60 bis 75 mol-%, neutralisiert.

Ein weiterer Gegenstand der vorliegenden Erfindung sind wasserabsorbierende Polymerpartikel, die nach dem erfindungsgemäßen Verfahren erhältlich sind.

Die gemäß dem erfindungsgemäßen Verfahren erhältlichen wasserabsorbierenden Polymerpartikel weisen eine Zentrifugenretentionskapazität (CRC) von typischerweise mindestens 20 g/g, vorzugsweise mindestens 25 g/g,

bevorzugt mindestens 30 g/g, besonders bevorzugt mindestens 35 g/g, ganz besonders bevorzugt mindestens 40 g/g, auf. Die Zentrifugenretentionskapazität (CRC) der wasserabsorbierenden Polymerpartikel beträgt üblicherweise weniger als 60 g/g.

Die gemäß dem erfindungsgemäßen Verfahren erhältlichen wasserabsorbierenden Polymerpartikel weisen eine Absorption unter einem Druck von 4,83 kPa (AUP0.7psi) von typischerweise mindestens 15 g/g, vorzugsweise von mindestens 20 g/g, besonders bevorzugt von mindestens 25, ganz besonders bevorzugt von mindestens 30 g/g, auf. Die Absorption unter einem Druck von 4,83 kPa (AUP0.7psi) der wasserabsorbierenden Polymerpartikel beträgt üblicherweise weniger als 50 g/g.

Die gemäß dem erfindungsgemäßen Verfahren erhältlichen wasserabsorbierenden Polymerpartikel weisen eine Permeabilität (SFC) von typischerweise mindestens 2 x $10^{-7}$ $cm^3s/g$, vorzugsweise mindestens 10 x $10^{-7}$ $cm^3s/g$, bevorzugt mindestens 30 x $10^{-7}$ $cm^3s/g$, besonders bevorzugt mindestens 60 x $10^{-7}$ $cm^3s/g$, ganz besonders bevorzugt mindestens 200 x $10^{-7}$ $cm^3s/g$, auf. Die Permeabilität (SFC) der wasserabsorbierenden Polymerpartikel beträgt üblicherweise weniger als 500 x $10^{-7}$ $cm^3s/g$.

Der mittlere Durchmesser der Polymerpartikel beträgt mindestens 200 µm, vorzugsweise von 250 bis 600 µm, besonders von 300 bis 500 µm, wobei der Partikeldurchmesser durch Lichtstreuung bestimmt werden kann und den volumengemittelten mittleren Durchmesser bedeutet. 90% der Polymerpartikel weisen einen Durchmesser von 100 bis 800 µm, vorzugsweise von 150 bis 700 µm, besonders bevorzugt von 200 bis 600 µm, auf.

Ein weiterer Gegenstand der vorliegenden Erfindung sind Verfahren zur Herstellung von Hygieneartikeln, insbesondere Windeln, umfassend die Verwendung gemäß obengenannten Verfahrens hergestellter wasserabsorbierender Polymerpartikel.

Ein weiterer Gegenstand der vorliegenden Erfindung ist die Verwendung erfindungsgemäßer wasserabsorbierender Polymerpartikel in Hygieneartikeln, zur Verdickung von Abfällen, insbesondere medizinischen Abfällen, oder als wasserrückhaltendes Mittel in der Landwirtschaft.

Die wasserabsorbierenden Polymerpartikel werden mittels der nachfolgend beschriebenen Testmethoden geprüft.

**Methoden:**

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymere werden vor der Messung gut durchmischt. mittlere Sphärizität (mSPHT)

Die mittlere Sphärizität (mSPHT) wird mit dem Bildanalysesystem Camsizer® (Retsch Technolgy GmbH; DE) bestimmt.

Zur Messung wird das Produkt über einen Trichter aufgegeben und mit einer Dosierrinne zum Fallschacht gefördert. Während die Partikel an einer Leuchtwand vorbeifallen werden sie wahlweise von einer Kamera erfasst. Die aufgenommenen Bilder werden von der Software entsprechend der ausgewählten Parameter ausgewertet.

Zur Charakterisierung der Rundheit wird die im Programm mit Sphärizität gekennzeichnete Messgröße herangezogen. Angegeben sind die mittleren, mit dem Volumen gewichteten Sphärizitäten, wobei das Volumen der Partikel über den Äquivalentdurchmesser $xc_{min}$ ermittelt werden. Zur Bestimmung des Äquivalentdurchmessers $xc_{min}$ wird der jeweils längste Sehnendurchmesser für insgesamt 32 unterschiedliche Raumrichtungen gemessen. Der Äquivalentdurchmesser $xc_{min}$ ist der kürzeste dieser 32 Sehnendurchmesser. Der Äquivalentdurchmesser $xc_{min}$ entspricht der Maschenweite eines Siebes, das das Partikel gerade noch passieren kann. Zur Erfassung der Partikel wird die sogenannte CCD-Zoom Kamera (CAM-Z) eingesetzt. Zur Steuerung der Dosierrinne wird ein Flächenbelegungsanteil von 0,5% vorgegeben.

**Wassergehalt**

Der Wassergehalt der wasserabsorbierenden Polymerpartikel wird gemäß der von der EDANA (European Disposables and Nonwovens Association) empfohlenen Testmethode Nr. WSP 230.2-05 "Moisture content" bestimmt.

**Zentrifugenretentionskapazität (CRC Centrifuge Retention Capacity)**

Die Zentrifugenretentionskapazität der wasserabsorbierenden Polymerpartikel wird gemäß der von der EDANA (European Disposables and Nonwovens Association) empfohlenen Testmethode Nr. WSP 241.2-05 "Centrifuge retention capacity" bestimmt.

**Absorption unter Druck (AUP0.7psi Absorption Under Pressure)**

Die Absorption unter Druck wird gemäß der von der EDANA (European Disposables and Nonwovens Association) empfohlenen Testmethode Nr. WSP 242.2-05 "Absorption under pressure" bestimmt, wobei ein Gewicht mit 49 $g/cm^2$ (0,7 psi) statt eines Gewichts mit 21 $g/cm^2$ (0,3 psi) verwendet wird.

**Flüssigkeitsweiterleitung (SFC Saline Flow Conductivity)**

Die Flüssigkeitsweiterleitung einer gequollenen Gelschicht unter Druckbelastung von 0,3 psi (2070 Pa) wird, wie in EP 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 640 330 A1. Der Durchfluss wird automatisch erfasst.

Die Flüssigkeitsweiterleitung (SFC) wird wie folgt berechnet:

$$SFC\ [cm^3s/g] = (Fg(t=0) \times L0)/(d \times A \times WP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in $g/cm^3$, A die Fläche der Gelschicht in $cm^2$ und WP der hydrostatische Druck über der Gelschicht in $dyn/cm^2$ darstellt.

**Absorptions-Index (AUP0.7psi-Index)**

Der Absorptions-Index(AUPO.7psi-Index) beschreibt die mechanische Stabilität der wasserabsorbierenden Polymerpartikel.

Dazu werden 20 g wasserabsorbierende Polymerpartikel in eine zylindrische Porzellanmühle mit einem Innenvolumen von ca. 360 ml eingewogen. Die Porzellanmühle hat eine Innenlänge von 8,8 cm und einen Innendurchmesser von 7,2 cm. Zusätzlich werden 24 zylindrische Porzellankörper eingefüllt. Die Porzellankörper haben eine Höhe von 1,25 cm und einen Durchmesser von 1,25 cm. Das Gewicht eines Porzellankörpers beträgt 5,3 g. Die zylindrische Porzellanmühle wird verschlossen und 15 Minuten mit 150 Umdrehungen pro Minute mittels eines Walzenantriebs gerollt.

Die Absorption unter Druck (AUP0.7psi) der wasserabsorbierenden Polymerpartikel wird vor und nach der mechanischen Belastung gemessen.

Der Absorptions-Index (AUP0.7psi-Index) wird wie folgt berechnet:

$$AUP0.7psi\text{-}Index = AUP0.7psi_{nachher}/AUP0.7psi_{vorher} \times 100\%$$

wobei $AUP0.7psi_{vorher}$ die Absorption unter Druck (AUP0.7psi) der Polymerpartikel vor der mechanischen Belastung und $AUP0.7psi_{nachher}$ die Absorption unter Druck (AUP0.7psi) der Polymerpartikel nach der mechanischen Belastung ist.

**Stabilitäts-Index**

Der Stabilitäts-Index beschreibt die mechanische Stabilität der wasserabsorbierenden Polymerpartikel.

Die mechanische Belastung wird wie bereits beim Absorptions-Index beschrieben durchgeführt.

Der Anteil wasserabsorbierender Polymerpartikel mit einer Partikelgröße von kleiner 100 µm wird vor und nach der mechanischen Belastung gemessen. Enthalten die wasserabsorbierenden Polymerpartikel über 1 Gew.-% Partikel mit einer Partikelgröße kleiner 100 µm, so sind diese vorher abzutrennen.

Der Stabilitäts-Index wird wie folgt berechnet:

$$Stabilit\ddot{a}ts\text{-}Index = (Partikel{<}100\mu m_{nachher} - Partikel{<}100\mu m_{vorher})/100\ Gew.\text{-}\%$$

wobei $Partikel{<}100\mu m_{vorher}$ der Gewichtsanteil an Polymerpartikeln mit einer Partikelgröße kleiner 100 µm vor der

mechanischen Belastung und Partikel<100µm$_{nachher}$ der Gewichtsanteil an Polymerpartikeln mit einer Partikelgröße kleiner 100 µm nach der mechanischen Belastung ist.

Die Partikel mit einer Partikelgröße von kleiner 100 µm werden photooptisch mit einem PartAn Particle Analyzer Typ 2001 F/L (Fa. AnaTec, Duisburg, DE) bestimmt. Zur Messung werden 20 g Polymerpartikel verwendet.

**Beispiele:**

**Beispiel 1**

14,3 kg Natriumacrylat (37,5 gew.-%ige Lösung in Wasser) und 1,6 kg Acrylsäure wurden mit 29 g 15-fach ethoxiliertem Trimethylolpropantriacrylat gemischt. Die Lösung wurde in einen erwärmten, mit Stickstoffatmosphäre gefüllten Vertropfungsturm vertropft (180°C, 12m Höhe, 2m Breite, Gasgeschwindigkeit 0,1 m/s im Gleichstrom). Die Dosiergeschwindigkeit betrug 16 kg/h. Die Vertropferplatte wies 30 Bohrungen ä 170 µm auf. Der Durchmesser der Vertropferplatte betrug 65 mm. Der Initiator wurde kurz vor dem Vertropfer über einen statischen Mischer mit der Monomerlösung gemischt.

Als Initiator wurde eine 6,5 gew.-%ige Lösung von 2,2'-Azobis[2-(2-imidazolin-2-yl)propan]dihydrochlorid in Wasser verwendet. Die Dosiergeschwindigkeit der Initiatorlösung betrug 0,44 kg/h. Die Gasaustrittstemperatur aus dem Vertropfungsturm betrug 126°C.

Anschließend wurden die erhaltenen wasserabsorbierenden Polymerpartikel nachvernetzt. Dazu wurden 200 g wasserabsorbierende Polymerpartikel in einer Küchenmaschine auf mittlerer Rührstufe mittels einer Zweistoffdüse mit 6,3 g Nachvernetzerlösung besprüht. Die Nachvernetzerlösung bestand aus 0,3 g 2-Hydroxyethyl-2-oxazolidon, 4,2 g Wasser und 1,8 g Isopropanol. Das feuchte Polymer wurde mit einem Spatel nochmals homogenisiert und in einem Umlufttrockenschrank 90 Minuten bei 170°C wärmebehandelt. Die nachvernetzten Polymerpartikel wurden über ein 850 µm Sieb von Klumpen befreit.

**Beispiel 2**

Die gemäß Beispiel 1 erhaltenen nachvernetzten wasserabsorbierenden Polymerpartikel wurden beschichtet. Dazu wurden 200 g nachvernetzte wasserabsorbierende Polymerpartikel im Wärmeschrank mindestens 30 Minuten bei 80°C vorgewärmt und in einer Küchenmaschine auf mittlerer Rührstufe mittels einer Zweistoffdüse mit dem Beschichtungsmittel besprüht. Als Beschichtungsmittel wurden 8,0 g Wasser eingesetzt. Es wurde eine Minute nachgerührt. Nach einer Lagerung von 16 Stunden wurden die beschichteten Polymerpartikel über ein 850 µm Sieb von Klumpen befreit.

Die Ergebnisse sind in Tabelle 1 zusammengefasst.

**Beispiel 3**

Es wurde verfahren wie unter Beispiel 2. Als Beschichtungsmittel wurde eine Lösung aus 8,0 g Wasser und 0,306 g Sorbitanmonolaurat eingesetzt.

Die Ergebnisse sind in Tabelle 1 zusammengefasst.

**Beispiel 4**

Es wurde verfahren wie unter Beispiel 2. Als Beschichtungsmittel wurde eine Lösung aus 8,0 g Wasser, 0,306 g Sorbitanmonolaurat und 3,0 g Triethanolamin eingesetzt.

Die Ergebnisse sind in Tabelle 1 zusammengefasst.

Beispiel 5

Es wurde verfahren wie unter Beispiel 2. Als Beschichtungsmittel wurde eine Lösung aus 8,0 g Wasser, 0,306 g Sorbitanmonolaurat und 6,0 g Triethanolamin eingesetzt.

Die Ergebnisse sind in Tabelle 1 zusammengefasst.

Tab. 1: Analysenergebnisse

| Bsp | AUP0.7psi (vorher) | AUP0.7psi (nachher) | AUP0.7psi-Index | Partikel<100µm (vorher) | Partikel<100µm (nachher) | Stabilitäts-Index |
|---|---|---|---|---|---|---|
| 1*) | 24,7 g/g | 12,8 g/g | 51,8% | 0,2 Gew.-% | 30,9 Gew.-% | 0,31 |

(fortgesetzt)

| Bsp | AUP0.7psi (vorher) | AUP0.7psi (nachher) | AUP0.7psi-Index | Partikel<100μm (vorher) | Partikel<100μm (nachher) | Stabilitäts-Index |
|---|---|---|---|---|---|---|
| 2 | 24,2 g/g | 16,4 g/g | 67,9% | 0,1 Gew.-% | 20,3 Gew.-% | 0,20 |
| 3 | 24,1 g/g | 19,0 g/g | 78,8% | 0,1 Gew.-% | 6,6 Gew.-% | 0,065 |
| 4 | 23,6 g/g | 18,4 g/g | 78,0% | 0,1 Gew.-% | 6,6 Gew.-% | 0,065 |
| 5 | 22,8 g/g | 19,4 g/g | 85,1% | 0,1 Gew.-% | 4,8 Gew.-% | 0,047 |
| *) Vergleichsbeispiel | | | | | | |

**Patentansprüche**

**1.** Verfahren zur Herstellung wasserabsorbierender Polymerpartikel durch Polymerisation von Tropfen einer Monomerlösung, enthaltend

a) mindestens ein ethylenisch ungesättigtes Monomer,
b) wahlweise mindestens einen Vernetzer,
c) mindestens einen Initiator,
d) Wasser,

in einer die Tropfen umgebenden Gasphase, wobei die erhaltenen Polymerpartikel nachvernetzt werden, **dadurch gekennzeichnet, dass** die nachvernetzten Polymerpartikel zumindest teilweise beschichtet werden.

**2.** Verfahren gemäß Anspruch 1, **dadurch gekennzeichnet, dass** die nachvernetzten Polymerpartikel mit Wasser, einer wässrigen Lösung, einem Alkanolamin, einem Polymeren und/oder einem Wachs beschichtet werden.

**3.** Verfahren gemäß Anspruch 2, **dadurch gekennzeichnet, dass** der Feststoffgehalt der Monomerlösung mindestens 35 Gew.-% beträgt.

**4.** Verfahren gemäß einem der Ansprüche 1 bis 3, **dadurch gekennzeichnet, dass** das Monomer a) zu mindestens 50 mol-% zumindest teilweise neutralisierte Acrylsäure ist.

**5.** Verfahren gemäß einem der Ansprüche 1 bis 4, **dadurch gekennzeichnet, dass** die Tropfen einen mittleren Durchmesser von mindestens 100 μm aufweisen.

**6.** Wasserabsorbierende Polymerpartikel, enthaltend mindestens einen Hohlraum im Partikelinneren, wobei die Vernetzungsdichte an der Partikeloberfläche erhöht wurde, die Polymerpartikel einen mittleren Durchmesser von mindestens 200 μm und 90% der Polymerpartikel einen Durchmesser von 100 bis 800 μm aufweisen, die Partikel eine mittlere Sphärizität von mindestens 0,84 und einen Stabilitäts-Index von weniger als 0,15 aufweisen.

**7.** Polymerpartikel gemäß Anspruch 6, **dadurch gekennzeichnet, dass** die Polymerpartikel mindestens einen Hohlraum aufweisen und das Verhältnis von maximalem Durchmesser des Hohlraumes zu maximalem Durchmesser des Polymerpartikels mindestens 0,1 beträgt.

**8.** Polymerpartikel gemäß einem der Ansprüche 6 oder 7, **dadurch gekennzeichnet, dass** die Polymerpartikel zu mindestens 50 mol-% zumindest teilweise neutralisierter polymerisierter Acrylsäure enthalten.

**9.** Polymerpartikel gemäß einem der Ansprüche 6 bis 8, **dadurch gekennzeichnet, dass** die Polymerpartikel eine Absorption unter Druck (AUP0.7psi) von mindestens 15 g/g aufweisen.

**10.** Verwendung der Polymerpartikel gemäß einem der Ansprüche 6 bis 9 zur Herstellung von Hygieneartikeln.

**11.** Hygieneartikel, enthaltend Polymerpartikel gemäß einem der Ansprüche 6 bis 9.

## Claims

1. A process for producing water-absorbing polymer particles by polymerizing droplets of a monomer solution comprising

    a) at least one ethylenically unsaturated monomer,
    b) optionally at least one crosslinker,
    c) at least one initiator,
    d) water,

    in a gas phase surrounding the droplets, the resulting polymer particles being postcrosslinked, wherein the postcrosslinked polymer particles are at least partly coated.

2. The process according to claim 1, wherein the postcrosslinked polymer particles are coated with water, an aqueous solution, an alkanolamine, a polymer and/or a wax.

3. The process according to claim 2, wherein the solids content of the monomer solution is at least 35% by weight.

4. The process according to any of claims 1 to 3, wherein the monomer a) is at least partly neutralized acrylic acid to an extent of at least 50 mol%.

5. The process according to any of claims 1 to 4, wherein the droplets have a mean diameter of at least 100 μm.

6. A water-absorbing polymer particle comprising at least one cavity in the particle interior, the crosslinking density at the particle surface having been increased, the polymer particle having a mean diameter of at least 200 μm and 90% of the polymer particle having a diameter of 100 to 800 μm, the particle having a mean sphericity of at least 0.84 and a stability index of less than 0.15.

7. The polymer particles according to claim 6, wherein the polymer particle has at least one cavity and wherein the ratio of maximum diameter of the cavity to maximum diameter of the polymer particle is at least 0.1.

8. The polymer particle according to either of claims 6 and 7, wherein the polymer particle comprises at least partly neutralized polymerized acrylic acid to an extent of at least 50 mol%.

9. The polymer particle according to any of claims 6 to 8, wherein the polymer particle has an absorption under pressure (AUP0.7psi) of at least 15 g/g.

10. The use of the polymer particles according to any of claims 6 to 9 for producing hygiene articles.

11. A hygiene article comprising polymer particles according to any of claims 6 to 9.

## Revendications

1. Procédé pour la préparation de particules polymères absorbant l'eau par polymérisation de gouttes d'une solution de monomères, contenant

    a) au moins un monomère éthyléniquement insaturé,
    b) au choix au moins un agent de réticulation,
    c) au moins un initiateur,
    d) de l'eau,

    dans une phase gazeuse entourant les gouttes, les particules polymères obtenues étant post-réticulées, **caractérisé en ce que** les particules polymères post-réticulées sont au moins partiellement revêtues.

2. Procédé selon la revendication 1, **caractérisé en ce que** les particules polymères post-réticulées sont revêtues avec de l'eau, une solution aqueuse, une alcanolamine, un polymère et/ou une cire.

3. Procédé selon la revendication 2, **caractérisé en ce que** la teneur en solides de la solution de monomères est d'au moins 35% en poids.

4. Procédé selon l'une quelconque des revendications 1 à 3, **caractérisé en ce que** le monomère a) est, à raison d'au moins 50% en mole, de l'acide acrylique au moins partiellement neutralisé.

5. Procédé selon l'une quelconque des revendications 1 à 4, **caractérisé en ce que** les gouttes présentent un diamètre moyen d'au moins 100 μm.

6. Particules polymères absorbant l'eau, contenant au moins un espace creux à l'intérieur des particules, la densité de réticulation à la surface des particules étant augmentée, les particules polymères présentant un diamètre moyen d'au moins 200 μm et 90% des particules polymères présentant un diamètre de 100 à 800 μm, les particules présentant une sphéricité moyenne d'au moins 0,84 et un indice de stabilité de moins de 0,15.

7. Particules polymères selon la revendication 6, **caractérisées en ce que** les particules polymères présentent au moins un espace creux et le rapport du diamètre maximal de l'espace creux au diamètre maximal de la particule polymère est d'au moins 0,1.

8. Particules polymères selon l'une quelconque des revendications 6 ou 7, **caractérisées en ce que** les particules polymères contiennent à raison d'au moins 50% en mole de l'acide acrylique polymérisé au moins partiellement neutralisé.

9. Particules polymères selon l'une quelconque des revendications 6 à 8, **caractérisées en ce que** les particules polymères présentent une absorption sous pression (AUP0,7 psi) d'au moins 15 g/g.

10. Utilisation des particules polymères selon l'une quelconque des revendications 6 à 9 pour la production d'articles hygiéniques.

11. Articles hygiéniques contenant des particules polymères selon l'une quelconque des revendications 6 à 9.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 348180 A1
- WO 9640427 A1
- US 5269980 A
- DE 10314466 A1
- DE 10340253 A1
- DE 102004024437 A1
- DE 102005002412 A1
- DE 102006001596
- DE 102004042946 A1
- DE 102004042948 A1
- DE 102004042955 A1
- DE 102005019398 A1
- WO 2006079631 A1
- EP 703265 A1
- EP 755964 A2
- EP 530438 A1
- EP 547847 A1
- EP 559476 A
- EP A1 A
- EP 632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 200232962 A2
- EP 343427 A2
- DE 4308842 A1
- EP 83022 A2
- EP 543303 A1
- EP 937736 A2
- DE 3314019 A1
- DE 3523617 A1
- EP 450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 200331482 A1
- DE 3713601 A1
- EP 640330 A1


### In der Beschreibung aufgeführte Nicht-Patentliteratur


- **F.L. BUCHHOLZ ; A.T. GRAHAM.** Modern Superabsorbent Polymer Technology. Wiley-VCH, 1998, 71-103
- **HOUBEN-WEYL.** Methoden der Organischen Chemie. vol. E20/2, 1561-1721
- **HOUBEN-WEYL.** Methoden der Organischen Chemie. vol. E20/2, 1721-1752
- Chemie und Technologie des kalthärtenden Siliconkautschuks. SILICONE - Chemie und Technologie. VULKAN-VERLAG, 28. April 1989, 49-64
- *Plaste Kautsch,* 1978, vol. 25, 440-444
- **HOUBEN-WEYL.** Methoden der Organischen Chemie. vol. E20/2, 1404-1429
- **HOUBEN-WEYL.** Methoden der Organischen Chemie. vol. 14/2, 462-552
- *METHODEN DER ORGANISCHEN CHEMIE,* vol. Band E20/2, 1891-1994
- **HOUBEN-WEYL.** Methoden der Organischen Chemie. vol. E 20/2, 1443-1457
- DGF-Einheitsmethoden: Untersuchung von Fetten, Fettprodukten und verwandten Stoffen. **ABTEILUNG M.** Wachse und Wachsprodukte. Wissenschaftliche Verlagsgesellschaft, 1975